Europäisches Patentamt
European Patent Office
Office européen des brevets

(11) Publication number: **0 253 595 A2**

# EUROPEAN PATENT APPLICATION

(21) Application number: 87306134.5

(22) Date of filing: 10.07.87

(51) Int. Cl.[4]: C07D 207/333 , H01B 1/12 , C25B 3/10

Claims for the following Contracting States: AT + ES.

(30) Priority: 16.07.86 GB 8617358
18.11.86 GB 8627565

(43) Date of publication of application: 20.01.88 Bulletin 88/03

(84) Designated Contracting States: AT BE CH DE ES FR GB GR IT LI LU NL SE

(71) Applicant: COOKSON GROUP plc
14 Gresham Street
London EC2V 7AT(GB)

(72) Inventor: Kathirgamanathan, Poopathy
14 Sandhurst Avenue
North Harrow Middlesex, HA2 7AT(GB)
Inventor: Smith, Nigel Robert Martin
8 Normanton Road Packington
Nr. Ashby Leicestershire, LE6 5WR(GB)

(74) Representative: Allard, Susan Joyce et al
BOULT, WADE & TENNANT 27 Furnival Street
London EC4A 1PQ(GB)

(54) **Novel pyrrole monomers and polymers prepared therefrom.**

(57) Novel pyrroles of the general formula

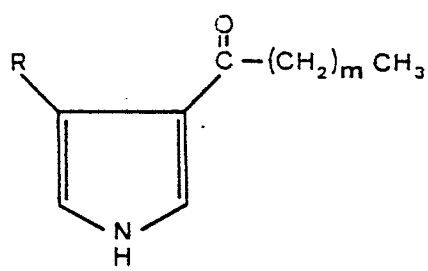

wherein m is an integer of from 1 to 18 and R is certain specified substituent groups can be polymerised to form hompolymers or copolymers which are electrically conducting. The polymers are of particular use in photovoltaic cells, such as solar cells.

EP 0 253 595 A2

## NOVEL PYRROLE MONOMERS AND POLYMERS PREPARED THEREFROM

The present invention relates to certain novel pyrrole monomers and to electrically conducting polymers which are prepared therefrom.

EP-0203438-A published on 3rd December, 1986 discloses certain poly(substituted heterocycles) and solution comprising these polymers either in the conductive or non-conductive form. The general structure of the polymers is given in this specification as

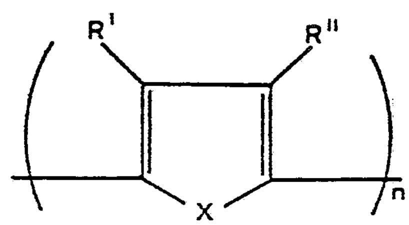

where n is a natural number,
R′ and R″ are various substituent groups, including alkyl, alkenyl, alkoxy and alkanoyl, and
X includes O, S and NH.

The specification does not contain therein any examples of polypyrroles or disclose the preparation of pyrrole monomers. The specification suggests that the polymer solutions are useful for casting conductive films, coatings and other conductive polymer articles.

We have now surprisingly found that a group of pyrrole polymers, which fall generally within the scope of polymers as defined in EP-0203438-A, have exceptional properties with regard to their use in photovoltaic cells.

Accordingly, the present invention provides a pyrrole having the general formula:

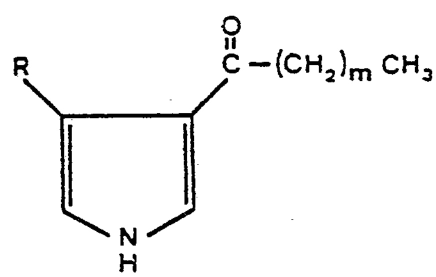

wherein m is an integer of from 1 to 18
and R is hydrogen, an alkyl group
containing from 1 to 6 carbon atoms, an aryl group, or is the group

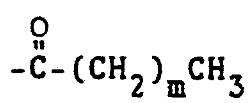

where m is as above defined.

Preferred pyrrole monomers are those in which R is a hydrogen atom. Particularly preferred pyrrole monomers are 3-octanoylpyrrole, 3-dodecanoylpyrrole and 3-octadecanoylpyrrole.

The pyrroles of the present invention may be synthesised according to the following preferred reaction - scheme:-

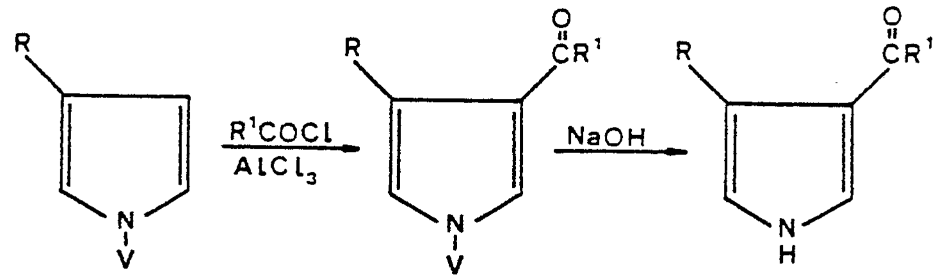


In the above reaction scheme the group R is as above defined, V represents a protecting group and the group $R^1$ corresponds to the side chain $-(CH_2)_mCH_3$ attached to the keto group. The preferred protecting group V is a p-toluenesulphonyl group (tosyl), although any other N-protecting group may also be used, for example acetyl.

The pyrrole derivatives of the present invention can be polymerised, for example using an electrochemical polymerization process, to provide electroconductive polymers.

The present invention includes within its scope a process for the preparation of a polypyrrole derivative which process comprises subjecting a pyrrole monomer as above defined in a non-aqueous solvent to electrochemical oxidation at an electrode potential which is at least as electropositive as the oxidation potential of the pyrrole monomer.

The electrochemical polymerization of the monomers of the invention may be carried out, for example, in a single compartment cell using an anode of platinium, indium-tin oxide (ITO), tungsten, titanium, niobium, nickel, lead or graphite.

The present invention furthermore includes within its scope a polypyrrole derivative which comprises repeating units of the general formula:

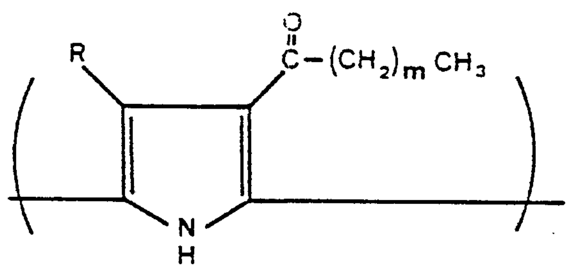

where R and m as defined above, and counterions of the formula X, where X is chloride, bromide, sulphate, bisulphate, nitrate, tetrafluoroborate, alkylsulphonate, arylsulphonate, arenecarboxylate, alkylcarboxylate, arenedicarboxylate, polystyrenesulphonate, polyacrylate, cellulose sulphonate, cellulose sulphate, anthracene sulphonate,
$H_2PO_3^-$ , $H_2PO_4^-$ , $PF_6^-$ , $SbF_6^-$ ,
$AsF_6^-$ or a perfluorinated polyanion.

The electrolyte is chosen so as to provide the counterions as above defined.

The solvent is preferably acetonitrile, dichloromethane, chloroform, nitromethane, nitrobenzene, propylene carbonate, N-methylpyrrolidone, sulpholane, dimethylformamide, dimethylsulphoxide or trichlorobenzene. The solvent may be used alone or as a mixture of two or more thereof.

The pyrrole polymers of the present invention are conductive and therefore may be used in thin film technology, as EMI/RF shielding materials, as antistatics, in electrochromic display systems, as ion and pH sensors, as battery electrode materials, as protective coatings for electrodes and as electrodes for the selective deposition of metal ions. The main application of these polymers is however in photovoltaic cells. The polymers are transparent and off white in colour and possess a band gap of about 3eV. The polymers can therefore be used as a p-type window material on an n-type absorber (e.g. n-CdTe) in photovoltaic cells, such as solar cells.

The pyrrole derivatives of the present invention may also be co-polymerised with other monomers, for example with pyrrole or any other monomer with which it will co-polymerise. These copolymers are also included within the scope of the invention.

The present invention will be further described with reference to the following Examples.

Example 1

Preparation of 3-octanoylpyrrole (1).

Octanoyl chloride (2.18 g, 13.4 mmol) was added dropwise to a suspension of aluminium trichloride (1.95 g, 14.6 mmol) in methylene chloride (25 ml) and the mixture stirred at 20°C for 10 minutes. A solution of N-tosylpyrrole (2.7 g, 12.2 mmol) [prepared as described by E.P. Papdopoulose and N.F. Haidar, Tetrahedron Lett., 1968, 1721] in methylene chloride (5 ml) was then added dropwise to the cooled (5°C) reaction mixture and stirring continued for 2 hours at 20°C. The reaction mixture was then quenched by pouring into iced water and the organic layer separated, dried with magnesium sulphate and evaporated in vacuo (40°C/$10^{-2}$ mm) to yield N-tosyl-3-octanoylpyrrole as an oil which crystallised on standing. This material was used without further purification for the next step. Yield was 3.3 g, 78% based on tosylpyrrole.

A mixtureof N-tosyl-3-octanoylpyrrole (1.5 g 4.32 mmol), dioxan (25 ml) and 5M sodium hydroxide solution (25 ml) was refluxed for 17 hours. After cooling to room temperature, the organic layer was separated and the aqueous layer extracted with ethyl acetate (3 x 50 ml). The combined organic phases were shaken with concentration brine solution, separated, dried with sodium sulphate, and the solvent evaporated in vacuo to leave an oil which slowly crystallised. Recrystallisation from hexane afforded 3-octanoylpyrrole (0.59 g, 66% based on N-tosyl-3-octanoylpyrrole). M.p. 71-72°C. (Found: C, 75.1; H, 10.3; N, 7.3. Calculated for $C_{12}H_{19}NO$: C, 74.6; H, 9.8; N, 7.7%); m/e Intensity (%): 193 (6, $M^+$), 122 (8,$C_7H_8NO$), 109 (74, $C_6H_7NO$), 94 (100, $C_5H_4NO$); δH($CDCl_3$): 0.87 (3H, t), 1.29 (8H, m), 1.71 (2H, t), 2.76 (2H, t). 6.65 (1H, dd), 6.78 (1H, dd), 7.44 (1H, dd), 9.43 (1H, br,s); δC($CDCl_3$): 14.0, 22.6, 25.2, 29.1, 29.5, 31.7, 39.8, 108.7, 119.5, 123.2, 126.0, 196.2.

Example 2

Preparation of 3-dodecanoylpyrrole (2)

Using the same method and reaction conditions as described above in Example 1,N-tosylpyrrole (7.0 g, 0.032 mol) in methylene chloride (35 ml), aluminium chloride (5.12 g, 0.038 mol) and dodecanoyl chloride (7.9 ml, 0.034 mol) in methylene chloride (60 ml) gave N-tosyl-3-dodecanoylpyrrole as an oil which crystallised on standing (10.5 g, 81% based on N-tosylpyrrole). M.p. 55-6°C. (Found: C 69.0; H, 8.5; N, 3.9. Calculated for $C_{23}H_{33}NO_3S$: C, 68.5; H, 8.2; N, 3.5%).

This compound was then detosylated to give 3-dodecanoylpyrrole (6.1 g, 94% based on N-tosyl-3-dodecanoylpyrrole). M.p. 62-3°C; (Found: C, 77.3; H, 11.0; N, 5.2. Calculated for $C_{16}H_{27}NO$; C, 77.1 H, 10.8; N, 5.6%); m/e Intensity (%): 249 (6, $M^+$), 122 (11, $C_7H_8NO$), 109 (90, $C_6H_7NO$), 94 (100, $C_5H_4NO$); δH($CDCl_3$): 0.87 (3H, t), 1.30 (16H, m), 1.71 (2H, m), 2.76 (2H, t), 6.65 (1H, dd), 6.78 (1H, dd), 7.44 (1H, dd), 9.48 (1H, br,s); δC($CDCl_3$): 14.0, 22.7. 25.2, 29.3, 29.5, 29.6, 31.6, 39.8, 108.6, 119.5, 123.3, 126.1, 197.6 ppm.

Example 3

Preparation of 3-octadecanoylpyrrole, (3)

Using the same method and reaction conditions as described above in Example 1, N-tosylpyrrole (7.5 g, 0.034 mol) in methylene chloride (50 ml), aluminium chloride (5.4 g, 0.040 mol) and octadecanoyl chloride (12.55 ml, 0.037 mol) in methylene chloride (100 ml) gave N-tosyl-3-octadecanoylpyrrole as an oil which crystallised on standing (14.7 g, 88% based on N-tosylpyrrole). M.p. 64-5°C; (Found: C, 71.0 H, 9.4; N, 3.3; Calculated for $C_{29}H_{45}NO_3S$; C, 71.4; H, 9.2; N, 2.9%).

This compound was then detosylated to give 3-octadecanoylpyrrole (7.4 g, 74% based on N-tosyl-3-octadecanoylpyrrole). M.p. 84-85°C (Found: C, 79.3; H, 11.8; N, 4.4. Calculated for $C_{22}H_{39}NO$: C, 79.3; H, 11.7; N, 4.2%); m/e Intensity (%); 333 (10, $M^+$), 122 (12, $C_7H_8NO$), 109 (100, $C_6H_7NO$), 94, (67, $C_5H_4NO$); δH-($CDCl_3$): 0.88 (3H, t), 1.25 (28H, m), 1.71 (2H, m), 2.76 (2H, t), 6.64 (1H, m), 6.77 (1H, d), 7.44 (1H, m), 9.88 (1H, br,s); δC($CDCl_3$): 14.1, 22.7, 25.3, 29.5, 29.7, 31.7, 39.8, 108.5, 119.7, 123.6, 125.6 and 197.7 ppm.

Example 4

Electrochemical polymerization of pyrrole monomers.

The electrochemical polymerization of pyrrole monomers was carried out in a single compartment electrochemical cell at a temperature of 10°C under an atmosphere of nitrogen using tetrabutylammonium hexafluorophosphate as the supporting electrolyte. The anode was either platinum of indium-tin oxide (ITO). Direct current conductivity measurements of the polymer films were obtained (using a four-probe technique) with a free-standing polymer film that had been peeled off the anode.

The polymerization conditions, which were as detailed above unless otherwise stated, are given in Table 1 below.

**TABLE 1**

| Compound Number | Concn. of Monomer (M) | Concn. of $Bu_4NPF_6$ (M) | Solvent | Condition | Current Density mA $cm^{-2}$ | No. of Coulombs |
|---|---|---|---|---|---|---|
| 1 | $2.2 \times 10^{-2}$ | $2.5 \times 10^{-3}$ | Aceto nitrile/ Dichloro- methane (1:1) (25ml) | $10^{o}C$ Nitrogen Atmos- phere | 0.5 + 0.1 ITO | 0.60 |
| 2 | $1.61 \times 10^{-2}$ | $2.6 \times 10^{-3}$ | Aceto- nitrile/ Dichloro- methane (1:1) (25ml) | do | 0.5 + 0.1 ITO | 0.58 |
| 3 | $4.81 \times 10^{-3}$ | $1.03 \times 10^{-3}$ | Aceto- nitrile/ Dichloro- methane (1:1) (25 ml) | do | 1.8 + 0.2 ITO | 1.62 |

The conductivities of some of these polymers are given in Table 2 below:

In both the Tables 1 and 2 the number allocated to the compounds are those used throughout the Specification.

The conductivity measurements for the compounds detailed in Table 2 below were carried out by the four-probe method. This was performed by four pressure contacts using a Keithley 228 Voltage/ Current source or a Thurlby 30V-2A Voltage/Current source. The current was measured with a Keithley 160 digital multimeter and voltage monitored by a Keithley 195A digital multimeter.

**TABLE 2**

| Compound | $\sigma$/S $cm^{-1}$ |
|---|---|
| 2 | $3.6 \times 10^2$ |
| 3 | 9.5 |

Example 5

A mixture of 25 ml acetonitrile and 25 ml dichloromethane containing 0.04 g of 3-octadecanoylpyrrole and 0.01g of tetrabutylammonium hexafluorophosphate was electrolysed at an ITO electrode at varying current densities for varying periods of time. A transparent conducting polymer was formed on the electrode. The results are given in Table 3 below.

**TABLE 3**

| Experiment Number | Current Density ($mA/cm^2$) | Time (minutes) | $\rho_s/S^{-1}$ □$^{-1}$ |
|---|---|---|---|
| 1 | 2.0 | 30 | $1.36 \times 10^4$ |
| 2 | 0.25 | 45 | $4.31 \times 10^5$ |
| 3 | 2.0 | 60 | $5.66 \times 10^3$ |
| 4 | 0.75 | 60 | $3.30 \times 10^4$ |

The polymers had a transmittance of greater than 60% in the range of 400 to 2000nm.

The volume conductivity of the sample used in Experiment 3 was 1.8 S $cm^{-1}$.

Inspection of the transmittance spectrum for the polymers indicates that there is a band gap ($\Delta Eg$) of approximately 3eV. Theremoelectric measurements indicate that these polymers are p-type semiconductors. They are also very lightly coloured and could therefore be used a p-type window materials on n-type absorbers in photovoltaic cells.

The polymers are, as expected, also photoconductive.

**Claims**

1. A pyrrole having the general formula

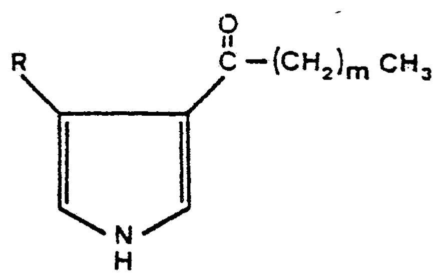

wherein m is an integer of from 1 to 18
and R is hydrogen, an alkyl group
containing from 1 to 6 carbon atoms, an aryl group, or is the group

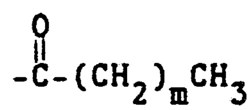

where m is as above defined.

2. A pyrrole as claimed in claim 1 wherein R is a hydrogen atom
3. 3-Octanoylpyrrole.
4. 3-Dodecanoylpyrrole.
5. 3-Octadecanoylpyrrole.
6. A process for the preparation of a pyrrole as claimed in claim 1, which process comprises reacting a pyrrole derivative of the formula

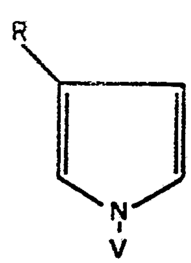

where R is as defined in claim 1 and V is a protecting group, preferably a p-toluenesulphonyl group, with an acid chloride of the formula $R^1COCl$ where $R^1$ is the group $-(CH_2)_mCH_3$ where m is as defined in claim 1, in the presence of an aluminium chloride catalyst to form the compound

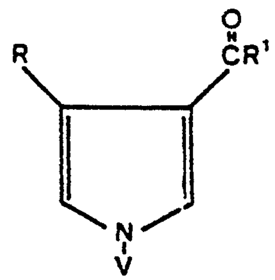

and subsequently removing the protecting group therefrom to give the compound of the formula

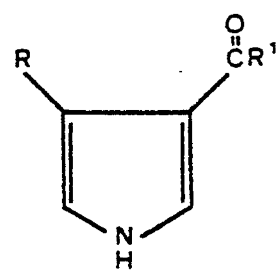

7. A polypyrrole derivative which comprises repeating units of the general formula

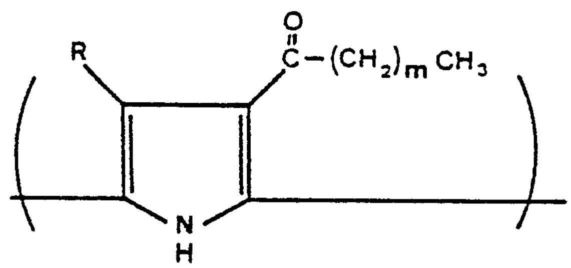

where R and m are as defined in claim 1 and counterions of the formula X, where X is chloride, bromide, sulphate, bisulphate, nitrate, tetrafluoroborate, alkylsulphonate, arylsulphonate, arenecarboxylate, alkylcarboxylate, arenedicarboxylate, polystyrenesulphonate, polyacrylate, cellulose sulphonate, cellulose sulphate, anthracene sulphonate, $H_2PO_3^-$, $H_2PO_4^-$, $PF_6^-$, $SbF_6^-$, $AsF_6^-$ or a perfluorinated polyanion.

8. A polypyrrole derivative as claimed in claim 7 wherein the counterion is $PF_6^-$.

9. A process for the preparation of a polypyrrole derivative as claimed in claim 7 or claim 8 which process comprises subjecting a pyrrole monomer as claimed in claim 1 in a non-aqueous solvent to electrochemical oxidation at an electrode potential which is at least as electropositive as the oxidation potential of the pyrrole monomer.

10. A process as claimed in claim 10 wherein the solvent is acetonitrile, dichloromethane, chloroform, nitromethane, nitrobenzene, propylene carbonate, N-methylpyrrolidone, sulpholane, dimethylformamide, dimethylsulphoxide or trichlorobenzene, or a mixture of two or more thereof.

11. A process as claimed in claim 9 or claim 10 wherein the electrochemical oxidation is carried out in an electrolyte which provides the counterion X, where X is as defined in claim 7.

12. A copolymer of a pyrrole derivative as claimed in any one of claims 1 to 6 with another monomer which is polymerisable therewith.

13. A copolymer as claimed in claim 12 wherein the other monomer is pyrrole.

14. The use of a polymer as claimed in claim 7 or claim 8 in a photovoltaic cell, preferably a solar cell.

Claims:

1. A process for the preparation of a pyrrole having the general formula

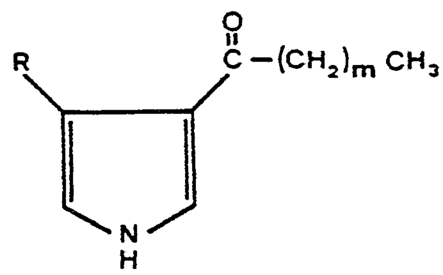

wherein m is an integer of from 1 to 18 and R is hydrogen, an alkyl group containing from 1 to 6 carbon atoms, an aryl group, or is the group

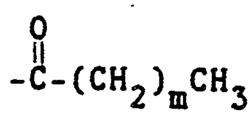

where m is as above defined, which process comprises reacting a pyrrole derivative of the formula

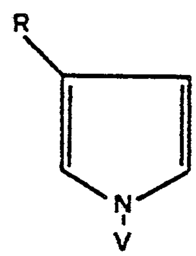

where R is as above defined and V is a protecting group, with an acid chloride of the formula $R^1COCl$ where $R^1$ is the group $-(CH_2)_mCH_3$ where m is as defined above in the presence of an aluminium chloride catalyst to form the compound

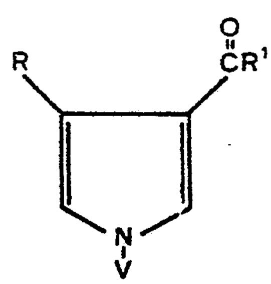

and subsequently removing the protecting group therefrom to give the compound of the formula

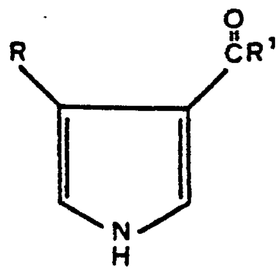

2. A process as claimed in claim 1 wherein the protecting group is a p-toluenesulphonyl group.

3. A process for the preparation of a polypyrrole derivative having repeating units of the general formula

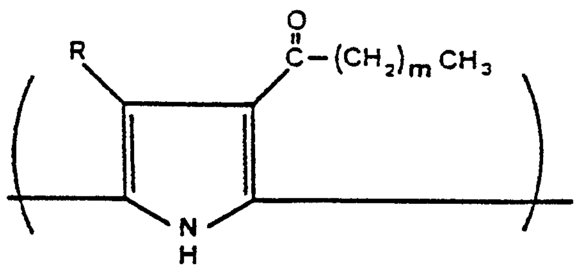

where R and m are as defined in claim 1 and counterions of the formula X, where X is chloride, bromide, sulphate, bisulphate, nitrate, tetrafluoroborate, alkylsulphonate, arylsulphonate, arenecarboxylate, alkylcarboxylate, arenedicarboxylate, polystyrenesulphonate, polyacrylate, cellulose sulphonate, cellulose sulphate, anthracene sulphonate, $H_2PO_3^-$ , $H_2PO_4^-$ , $PF_6^-$ , $SbF_6^-$ , $AsF_6^-$ or a perfluorinated polyanion, which process comprises subjecting a pyrrole monomer as defined in claim 1 in a non-aqueous solvent to electrochemical oxidation at an electrode potential which is at least as electropositive as the oxidation potential of the pyrrole monomer.

4. A process as claimed in claim 3 wherein the solvent is acetonitrile, dichloromethane, chloroform, nitromethane, nitrobenzene, propylene carbonate, N-methylpyrrolidone, sulpholane, dimethylformamide, dimethylsulphoxide or trichlorobenzene, or a mixture of two or more thereof.

5. A process as claimed in claim 3 or claim 4 wherein the electrochemical oxidation is carried out in an electrolyte which provides the counterion X.

6. The use of a polymer as defined in claim 3 in a photovoltaic cell, preferably a solar cell.